# EUROPÄISCHE PATENTSCHRIFT

(11) **EP 3 328 323 B1**
(45) Veröffentlichungstag und Bekanntmachung des Hinweises auf die Patenterteilung: **29.05.2019**
(21) Anmeldenummer: 16742309.4
(22) Anmeldetag: 25.07.2016
(51) Int. Cl.: A61F 2/54, A61F 2/78, A61F 2/80

(54) **PROTHESENSCHAFTHALTEVORRICHTUNG UND SYSTEM AUS PROTHESENSCHAFT UND PROTHESENSCHAFTHALTEVORRICHTUNG**
HOLDING DEVICE FOR A PROSTHETIC SOCKET AND SYSTEM CONSISTING OF A PROSTHETIC SOCKET AND A HOLDING DEVICE FOR A PROSTHETIC SOCKET
DISPOSITIF DE RETENUE D'EMBOÎTURE PROTHÉTIQUE ET SYSTÈME CONSTITUÉ D'UNE EMBOÎTURE PROTHÉTIQUE ET UN DISPOSITIF DE RETENUE D'EMBOÎTURE PROTHÉTIQUE

(30) Priorität: 29.07.2015 DE 102015112406
(43) Veröffentlichungstag der Anmeldung: 06.06.2018
(73) Patentinhaber: Otto Bock Healthcare Products GmbH, 1110 Wien (AT)
(72) Erfinder: WAGNER, Sonja, 1180 Wien (AT); FREY, Alice, 2000 Stockerau (AT); LUNZER, Walter, 1080 Wien (AT)
(74) Vertreter: Gramm, Lins & Partner Patent- und Rechtsanwälte PartGmbB
(86) Internationale Anmeldenummer: PCT/EP2016/067659
(87) Internationale Veröffentlichungsnummer: WO 2017/017057

(56) Entgegenhaltungen:
- GB-A- 206 333
- US-A- 1 366 453
- US-A- 1 718 095
- US-A- 2 592 842
- US-A- 3 188 655
- US-A- 4 258 441

## Beschreibung

Die Erfindung betrifft eine Prothesenschafthaltevorrichtung zur Sicherung eines Prothesenschaftes an einer oberen Extremität, mit einem Sicherungsteil, das an dem Torso unterhalb der Axilla der kontralateralen, unversorgten Seite eines Patienten entlangführbar ist, und mit einem Kopplungselement, das an dem Prothesenschaft festlegbar und mit dem Sicherungsteil verbindbar ist, sowie ein System aus Prothesenschaft und einer solchen Prothesenschafthaltevorrichtung. Die Prothesenschafthaltevorrichtung ist insbesondere zum Sichern von Oberarmschäften an einem Oberarmstumpf geeignet und vorgesehen.

Die US 2 592 842 A, die als nächstliegender Stand der Technik angesehen wird, betrifft ein Schultergurtgeschirr für einen elektrisch betriebenen, künstlichen Arm zu dessen Anbindung an einen Oberarmstumpf mit einem Oberarmschaft, der über einen Gurt und einen Halter an einem Oberkörpergurt festgelegt ist. Der Oberkörpergurt verläuft über die Schulter des versorgten Armes und unterhalb der unversorgten Achsel und ist über eine Rohrkonstruktion mit einem Hüftgurt gekoppelt. Die Rohrkonstruktion ist an einer Platte befestigt, die an dem Schultergurt festgelegt ist und an der der teleskopierbare Halter über ein Scharnier befestigt ist. Der Halter ist an dem Oberarmschaft verschwenkbar gelagert und mit dem Gurt verbunden.

Die US 5,403,268 A betrifft eine Orthese, mit der ausgekugelte Schultern unterstützt werden. Eine Manschette wird um den Oberarm angelegt, ein Schultergurt erstreckt sich von dem Schlüsselbein unterhalb der gegenüberliegenden Achsel um den Torso. Die Manschette ist über Riemen und Schnallen an dem Schultergurt befestigt. Eine solche Vorrichtung ist nicht für Prothesen vorgesehen.

Bei einem Verlust oder bei Fehlen des Unterarmes ist eine prothetische Versorgung notwendig, bei der ein Greifelement oder eine Prothesenhand an einem Unterarmelement befestigt ist. Innerhalb des Unterarmelementes können Antriebseinrichtungen, Steuereinrichtungen und Energiespeicher angeordnet sein. Zur Befestigung der Prothesenhand und des Unterarmteils werden diese Komponenten in der Regel gelenkig an einem Oberarmschaft gelagert, der an dem verbliebenen Oberarmstumpf des Patienten festgelegt wird. Die Festlegung eines Oberarmschaftes an dem Torso kann über aufwendige Riemenkonstruktionen erfolgen, bei denen die Riemen an dem Torso entlanggeführt werden. Riemen oder Gurte werden auf der kontralateralen Seite des Torsos entlanggeführt und mit dem Prothesenschaft befestigt, so dass der Oberarmschaft fest an dem Patienten gehalten ist.

Eine weitere Möglichkeit zur Festlegung einer Prothese an einem Oberarmstumpf besteht in der sogenannten Saugschafttechnologie, bei der ein Liner aus einem Kunststoff oder Silikonmaterial über den Oberarmstumpf gezogen wird. Der Prothesenschaft aus einem formstabilen Material dient als Aufnahme für den Liner mit dem Stumpf. Über mechanische Verriegelungsmittel oder Unterdruck wird der Liner an dem Schaft gehalten. Problematisch hierbei kann sein, dass die Haltekraft, die zwischen dem Liner und dem Stumpf aufgebracht wird, nicht ausreichen kann, um bei einer Zugbelastung die Prothese an dem Stumpf zu halten. Volumetrische Änderungen des Stumpfes können zu einer Reduzierung der Haftkraft führen. Darüber hinaus kann sich ein Unsicherheitsgefühl bei Patienten einstellen, wenn die Armprothese ausschließlich über den Liner an dem Stumpf gehalten wird.

Aufgabe der vorliegenden Erfindung ist es, eine Prothesenschafthaltevorrichtung und ein System aus Prothesenschaft und Prothesenschafthaltevorrichtung bereitzustellen, mit denen einerseits eine sichere Festlegung des Prothesenschaftes an dem Patienten gewährleistet und andererseits eine freie Beweglichkeit des Schultergelenkes ermöglicht wird.

Erfindungsgemäß wird diese Aufgabe durch eine Prothesenschafthaltevorrichtung mit den Merkmalen des Hauptanspruches sowie ein System aus einer Prothesenschafthaltevorrichtung und einem Prothesenschaft gemäß dem nebengeordneten Anspruch gelöst. Vorteilhafte Ausgestaltungen und Weiterbildungen der Erfindung sind in den Unteransprüchen, der Beschreibung sowie den Figuren offenbart.

Die Prothesenschafthaltevorrichtung zur Sicherung eines Prothesenschaftes an einer oberen Extremität, mit einem Sicherungsteil, das an dem Torso unterhalb der Axilla der kontralateralen, unversorgten Seite eines Patienten entlangführbar ist, und mit einem Kopplungselement, das an dem Prothesenschaft festlegbar und mit dem Sicherungsteil verbindbar ist, sieht vor, dass das Kopplungselement verschieblich an dem Sicherungsteil gelagert ist. Der Prothesenschaft, insbesondere in Ausgestaltung als Oberarmschaft, wird über ein Sicherungsteil an dem Oberkörper des Patienten fixiert. Das Sicherungsteil wird unterhalb der Axilla des Patienten auf der unversorgten Seite entlanggeführt und stellt dadurch sicher, dass Zugkräfte von der Prothesenschafthaltevorrichtung, die auf das Sicherungsteil ausgeübt werden, über den Torso und nicht über den Halsbereich aufgenommen werden. Das Kopplungselement ist an dem Prothesenschaft festgelegt oder festlegbar und verschieblich an dem Sicherungsteil gelagert, wodurch einerseits die mechanische Festlegung des Prothesenschaftes an dem Sicherungsteil gewährleistet ist und andererseits die freie Beweglichkeit im Schultergelenk der versorgten Seite unbeeinträchtigt bleibt. Der Oberarmknochen bewegt sich mit seinem Gelenkkopf in der Gelenkpfanne des Schulterblattes, wobei nur ein Viertel des Gelenkkopfes von der Pfanne umschlossen ist, was eine große Beweglichkeit des Schultergelenkes ermöglicht. Weiterhin ist eine Gelenkkapsel vorhanden, das ein Verstärkungsband aufweist, um den Gelenkkopf in der Gelenkpfanne zu halten. Weitere Bandführungen weist das Schultergelenk nicht auf, der Zusammenhalt des Gelenkes wird lediglich durch die Muskulatur bewirkt. Das Schultergelenk ist somit das beweglichste Gelenk, dessen Position an dem Rumpf zudem über das Schlüsselbein verlagerbar ist. Diese hohe Beweglichkeit führt unter anderem dazu, dass die herkömmliche Befestigung von Prothesenschäften an der Schulter mit Riemen und Schnallen zu Bewegungseinschränkungen führt, da diese Riemen die Beweglichkeit nicht ausgleichen können. Durch die verschiebliche Lagerung des Kopplungselementes an dem Sicherungsteil ist es möglich, einerseits die Unsicherheit der Patienten durch eine mechanische Festlegung zu verringern, ohne dass andererseits die Beweglichkeit eingeschränkt wird. Das Kopplungselement gleicht somit die Relativbewegung zwischen dem Sicherungsteil und dem Prothesenschaft aus, so dass ein physiologisches Mitschwingen des versorgten Armes ermöglicht wird.

Eine Weiterbildung der Erfindung sieht vor, dass das Kopplungselement lösbar an dem Prothesenschaft befestigbar ist. Die Lösbarkeit des Kopplungselementes hat den Vorteil, dass es unabhängig von dem Prothesenschaft gefertigt und angepasst werden kann. Die Reinigung des Kopplungselementes, gegebenenfalls mit den notwendigen Einrichtungen zur Festlegung an dem Prothesenschaft, kann unabhängig von dem Prothesenschaft erfolgen, beispielsweise durch Waschen.

Das Kopplungselement kann formschlüssig an dem Prothesenschaft befestigt sein, also unmittelbar an dem Prothesenschaft angeordnet sein, beispielsweise über Klettverschlüsse, Schnallen oder andere Verbindungselemente. Ebenfalls ist es möglich, bei einer einstückigen Ausgestaltung des Kopplungselementes eine trichterförmige Aufnahme vorzusehen, in der der Prothesenschaft eingeführt wird bzw. die um den Prothesenschaft herum angelegt wird, so dass ein Herausrutschen des Prothesenschaftes in distaler Richtung verhindert wird. Das Kopplungselement kann ebenfalls an einer Manschette oder Hülse angeordnet sein, die lösbar an dem Prothesenschaft befestigt oder befestigbar ist. Auf diese Weise ist ein leichtes Anlegen der Prothesenschafthaltevorrichtung durch den Prothesennutzer selbst möglich, da das Kopplungselement um den Prothesenschaft herum gelegt werden und über Riemen, Schnallen oder Klettverschlüsse daran befestigt werden kann. Ebenso ist es möglich, die Manschette oder Hülse mit einem geschlossenen Querschnitt auszugestalten, wobei das Kopplungselement dann an der Manschette ausgebildet, befestigt oder befestigbar ist.

Das Kopplungselement kann mehrteilig ausgebildet sein, beispielsweise aus einem Gurt und der oben erwähnten Manschette oder Hülse bestehen, die zusammen dann das Kopplungselement ausbilden. Alternativ ist das Kopplungselement ein Zugmittel oder Gurt, der über das Schlüsselbein in Richtung auf den Rücken in den Bereich der Brustwirbelsäule geführt, an einer Umlenkeinrichtung umgelenkt und dorsal zu dem Prothesenschaft geführt und dort festgelegt ist. Das Kopplungselement kann ein flexibles Zugelement aufweisen, beispielsweise einen Gurt, einen Riemen oder ein anderes Zugelement, das entweder unmittelbar an dem Prothesenschaft befestigt oder aber über die Manschette oder Hülse, die Teil des Kopplungselementes sein können, an dem Prothesenschaft festgelegt werden. Das Zugelement ist bevorzugt flexibel und unelastisch, um eine sichere Zuordnung des Prothesenschaftes zu dem Sicherungsteil zu gewährleisten. Das Zugelement kann beweglich, insbesondere verschieblich in einer an dem Sicherungsteil festgelegten oder ausgebildeten Führung geführt sein. Die Führung kann beispielsweise durch eine Ausnehmung in dem Sicherungsteil gebildet sein, alternativ kann die Führung als separates Bauteil an dem Sicherungsteil festgelegt sein, beispielsweise in Gestalt einer Schlaufe, eines Ringes oder eines ähnlichen Führungs, und Umlenkelement aus Metall oder Kunststoff, das einen vorzugsweise geschlossenen Hohlquerschnitt aufweist, um das Zugelement einerseits zu führen und andererseits eine Relativverlagerung des Zugelementes zu der Führung zuzulassen. Das Kopplungselement ist innerhalb der Führung frei beweglich, um die Schulterbewegung zu ermöglichen.

Das Kopplungselement kann in einer Schutzhülse oder auf einer Schutzauflage verschieblich geführt sein, um Reibung zwischen den Torso bzw. der Schulter und dem Kopplungselement zu vermeiden oder zu verhindern. Die Schutzhülse oder die Schutzauflage liegt im angelegten Zustand der Prothesenschafthaltevorrichtung auf dem Torso auf bzw. an dem Torso an, wobei die Prothesenschafthaltevorrichtung sowohl unmittelbar auf der Haut als auch oberhalb der Kleidung getragen werden kann. Durch die Schutzhülse oder Schutzauflage wird die Reibung zwischen dem Kopplungselement und dem darunter liegenden Gewebe, sei es Textil oder die Haut, vermieden.

Das Kopplungselement ist im angelegten Zustand bevorzugt auf der Vorderseite und der Rückseite der versorgten Schulter entlanggeführt, wobei der Bereich des Gelenkkopfes ausgespart bleiben kann. Ein vorderer Teil des Kopplungselementes erstreckt sich über das Schlüsselbein in Richtung auf das Schulterblatt, wird dort durch die Führung mit dem Sicherungsteil gekoppelt und dann im dorsalen Bereich der Axilla wieder in Richtung Prothesenschaft geführt. Über das Kopplungselement wird also die Vorderseite der Prothesenschafthaltevorrichtung oder des Prothesenschaftes mit der Rückseite der Prothesenschafthaltevorrichtung oder des Prothesenschaftes verbunden und dergestalt über die verschiebliche Lagerung und Umlenkung an dem Sicherungsteil gekoppelt, dass die jeweilige Vor- und Rückverlagerung des Armes und des Prothesenschaftes relativ zu dem Torso durch eine Verschiebung des Kopplungselementes ausgeglichen wird.

Das Sicherungsteil kann als flächiger Zuschnitt ausgebildet sein und eine Öffnung als Armdurchführung aufweisen. Alternativ kann das Sicherungsteil mit endseitigen Verbindungselementen ausgebildet sein, wodurch das Anlegen und das Individualisieren des Sicherungsteils erleichtert werden. Durch die Öffnung als Armdurchführung ist es möglich, dass das Sicherungsteil leicht angelegt werden kann, da der Arm der kontralateralen Seite einfach durch die Armdurchführung hindurchgeführt werden muss, um das Sicherungsteil anzulegen. Sind Verbindungselemente an dem Sicherungsteil vorgesehen, sind diese an Vorsprüngen oder Bereichen des Sicherungsteiles angeordnet, die miteinander verbunden werden können, so dass sich einer Öffnung ausbildet oder das Sicherungsteil um die Schulter herum angelegt werden kann, wobei ein Teil des Sicherungsteils unterhalb der Axilla entlanggeführt ist.

Das Sicherungsteil ist so ausgebildet, dass es im angelegten Zustand überwiegend dorsal geführt ist, also nur ein geringer Teil des Sicherungsteils frontal im Schulterbereich geführt wird, während der überwiegende Teil des Sicherungsteils dorsal positioniert ist. Im frontalen Schulterbereich ist das Sicherungsteil bevorzugt gurtartig ausgebildet, um die Beweglichkeit des Schultergelenks nicht oder nur möglichst gering zu beeinträchtigen. Im Bereich der Axilla kann das Sicherungsteil eine Polsterung aufweisen, beispielsweise in Gestalt einer Füllung mit perlenartigen Kunststoffen, die sich sehr gut der Anatomie des Anwenders anpassen sowie den Bewegungen des Armes folgen.

Eine Weiterbildung der Erfindung sieht vor, dass das Sicherungsteil im angelegten Zustand dorsal über die Medianebene in Richtung auf den Prothesenschaft hinausragt. Die Führung für das Kopplungselement ist ebenfalls im angelegten Zustand der Prothesenschafthaltevorrichtung dorsal positioniert, so dass keine Einschränkungen der Beweglichkeit im Brustbereich für den Patienten vorhanden sind.

Das Sicherungsteil und/oder das Kopplungselement können aus einem textilen Werkstoff, Abstandsgewirk und/oder Schaummaterial gefertigt sein, die ein leichtes Verarbeiten, ein leichtes Anlegen sowie eine gute Waschbarkeit ermöglichen.

Das Kopplungselement kann als Schlaufe ausgebildet oder geführt sein, wobei entweder eine direkte Verbindung mit dem Prothesenschaft oder eine Festlegung über einen Befestigungsabschnitt oder eine Befestigungsvorrichtung wie eine Hülse, Manschette, Spange oder dergleichen erfolgt.

Das erfindungsgemäße System besteht aus einem Prothesenschaft, insbesondere zur Aufnahme eines Oberarmstumpfes, sowie einer Prothesenschafthaltevorrichtung, wie sie oben beschrieben ist.

Nachfolgend werden Ausführungsbeispiele der Erfindung anhand der beigefügten Figuren näher erläutert. Es zeigen:
- Figur 1 -: eine angelegte Prothesenschafthaltevorrichtung in Rückansicht;
- Figur 2-: eine angelegte Prothesenschafthaltevorrichtung in Frontalansicht;
- Figur 3 -: eine angelegte Prothesenschafthaltevorrichtung in Seitenansicht;
- Figuren 4a - 4d -: Prothesenschafthaltevorrichtungen in unterschiedlichen Stellungen; sowie
- Figur 5 -: - Detaildarstellungen der Prothesenschafthaltevorrichtung

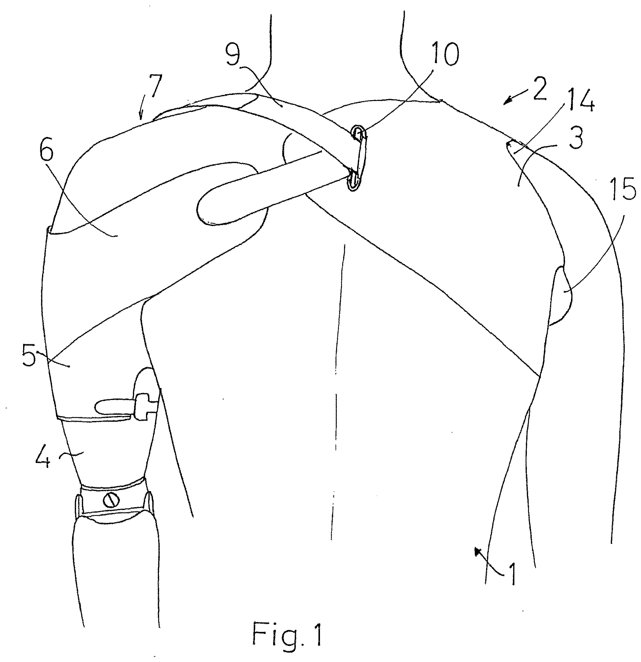

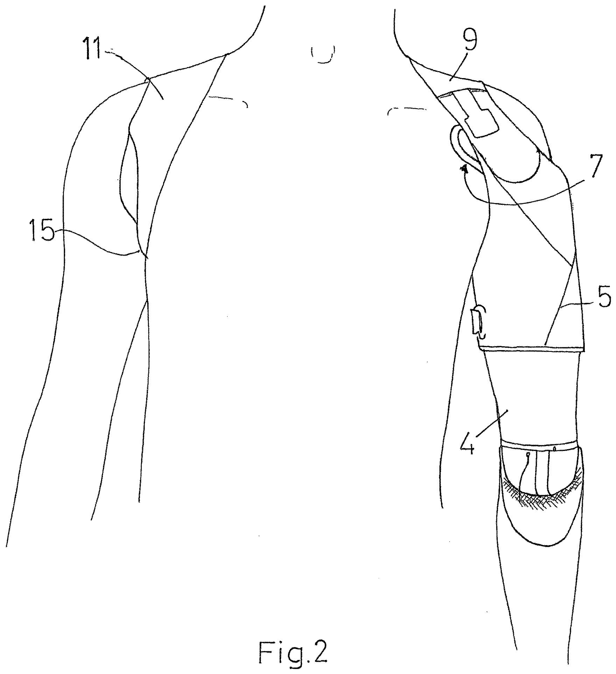

Figur 1 zeigt in einer Rückansicht eine Prothesenschafthaltevorrichtung, die an einem Patienten angelegt ist. Die Prothesenhaltevorrichtung weist ein Sicherungsteil 2 auf, das im dargestellten Ausführungsbeispiel aus einem textilen Grundkörper besteht, in dem eine Öffnung 14 als Armdurchführung ausgebildet ist. Dadurch wird das Sicherungsteil 2 im angelegten Zustand unterhalb der Axilla der unversorgten Seite entlanggeführt. Im Axilla-Bereich ist eine Polsterung 15 angeordnet, die als Polstermaterial Schaumstoff oder perlenartige Kunststoffe, beispielsweise aus Polystyrol aufweisen kann. Über die Polsterung 15 wird gewährleistet, dass die Blutzirkulation in diesem Bereich nicht behindert wird, zudem erfolgt eine Anpassung an die Anatomie des Anwenders, wodurch die Bewegung des unversorgten Armes nicht oder nur wenig eingeschränkt wird. Das Sicherungsteil 2 kann als einteiliger Grundkörper ausgebildet sein, der aus einem flächigen Material zugeschnitten ist. Es ist hinsichtlich der Passform vorteilhaft, wenn das Sicherungsteil 2 aus einem flächigen Zuschnitt hergestellt ist, wobei der Zuschnitt einen Rückenabschnitt 3 und einen riemenartigen oder gurtartigen Frontalabschnitt 11 aufweist, wobei der Frontalabschnitt 11 in der Figur 2 dargestellt ist. In dem Grundzuschnitt können der Rückenabschnitt 3 und der Frontalabschnitt 11 unverbundene Enden aufweisen, die vorteilhafterweise im Bereich der Axilla miteinander verbunden werden, beispielsweise vernäht werden, um eine an die Anatomie der Schulter angepasste Formgebung zu erreichen. Der Rückenabschnitt 3 ist wesentlich großflächiger als der Frontalabschnitt 11 und erstreckt sich im dargestellten Ausführungsbeispiel nach medial von dem Trapezmuskel über das Schulterblatt bis über die Medianebene hinaus und reicht lateral bis zum hinteren Axilla-Bereich. Durch das entsprechende Vernähen der beiden freien Enden im Axilla-Bereich ergibt sich die Öffnung 14 als Armdurchführung, so dass im angelegten Zustand das Sicherungsteil 2 großflächig im Rückenbereich anliegt, der Frontalabschnitt über das Schlüsselbein unter die Axilla verläuft und somit das Sicherungsteil 2 sicher an dem Torso 1 des Patienten festgelegt ist.

Der Rückenabschnitt 3 erstreckt sich über die Medianebene hinaus in Richtung auf die versorgte obere Extremität, bei der an einem Oberarmstumpf ein Prothesenschaft 4 festgelegt ist. Die Festlegung kann beispielsweise über einen Prothesenliner erfolgen, der über den nicht dargestellten Oberarmstumpf gezogen wird. Der Prothesenliner kann an dem Prothesenschaft 4 mechanisch oder durch Unterdruck festgehalten werden.

An der Außenseite des Prothesenschaftes 4 ist eine Hülse 5 oder Manschette angeordnet, die über einen Gurt oder ein anderes Befestigungselement eng um die Außenseite des Prothesenschaftes 4 herum angelegt ist. Sowohl der Prothesenschaft 4 als auch die Hülse 5 sind in proximaler Richtung konisch erweitert, wobei das distale Ende der Hülse 5 einen geringeren Umfang als das proximale Ende des Prothesenschaftes 4 aufweist, so dass der Prothesenschaft 4 nicht durch die distale Öffnung der Hülse 5 hindurch bewegt werden kann. Auf der Innenseite der Hülse 5 kann auch eine Beschichtung angeordnet sein, die rutschhemmend ist, so dass die einmal angelegte Hülse 5 fest an dem Prothesenschaft 4 fixiert ist. Die Hülse 5 kann auch mit einem offenen Querschnitt ausgebildet sein, also eher in Gestalt einer Manschette, wobei der offene Querschnitt über Verschlusseinrichtungen, beispielsweise Klettverschlüsse, Riemen oder andere Formschlusselemente geschlossen werden kann.

An der Hülse 5 ist, wie in der Figur 1 dargestellt, ein dorsaler Fortsatz 6 befestigt oder ausgebildet, der sich in medialer Richtung auf das Sicherungsteil 2 hin erstreckt. Der dorsale Fortsatz 6 verläuft im Wesentlichen über das Schulterblatt in Richtung auf den Nackenbereich des Patienten. An der Vorderseite der Hülse 5 ist, wie in Figur 2 dargestellt, ein frontaler Fortsatz 7 befestigt oder ausgebildet, der sich im vorderen Schulterbereich in Richtung auf das Schlüsselbein der versorgten Seite erstreckt. An dem vorderen Fortsatz 7 sowie dem dorsalen Fortsatz 6 ist ein Kopplungselement 9 in Gestalt eines flexiblen, bevorzugt unelastischen Gurtes befestigt. Das Kopplungselement 9 verläuft über das Schlüsselbein auf der versorgten Seite in Richtung auf eine Führung 10, die an dem Sicherungsteil 2 in Gestalt einer Brücke, eines Steges, eines Ringes oder einer Schlaufe ausgebildet ist. Das Kopplungselement 9 verläuft um die Führung 10 herum, wird also nur umgelenkt und in der Verschieblichkeit relativ zu der Führung nicht beeinträchtigt, und ist an dem dorsalen Fortsatz 6 festgelegt, beispielsweise festgenäht oder über ein Klettverschluss reversibel fixiert. Das Kopplungselement 9 ist verschieblich an der Führung 10 gelagert, das heißt, dass das Kopplungselement nicht an der Führung 10 festgelegt ist, sondern in ihr beweglich gelagert ist. Je nach Zugrichtung kann das Kopplungselement 9 in die eine oder andere Richtung bewegt werden.

Die Führung 10 ist im dargestellten Ausführungsbeispiel im Bereich der Wirbelsäule angeordnet, also auf der Medianebene, wenn die Prothesenschafthaltevorrichtung angelegt ist.

Bei einer lösbaren Befestigung des Kopplungselementes 9 an dem dorsalen Fortsatz 6 oder dem frontalen Fortsatz 7 ist es möglich, die effektive Länge des Kopplungselementes 9 einzustellen und die Prothesenschafthaltevorrichtung individuell an den jeweiligen Patienten anzupassen.

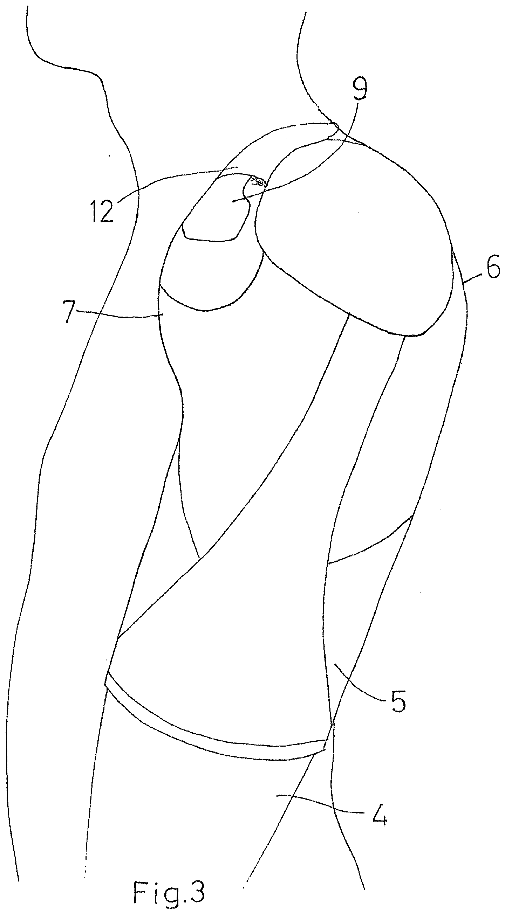

Figur 3 zeigt die angelegte Prothesenschafthaltevorrichtung in einer Seitenansicht. Es ist zu erkennen, dass der dorsale Fortsatz 6 und der frontale Fortsatz 7 das Schultergelenk dorsal und frontal umfassen, das Schultergelenk selbst jedoch freilassen und über die Hülse 5 den Prothesenschaft 4 sichern. Das Kopplungselement 9 ist im dargestellten Ausführungsbeispiel in einer Schutzhülse 12 geführt, die im Nackenbereich an den Patienten anliegt, um bei einer Bewegung des versorgten Armes ein unmittelbares Scheuern des Kopplungselementes 9 auf der Haut zu vermeiden.

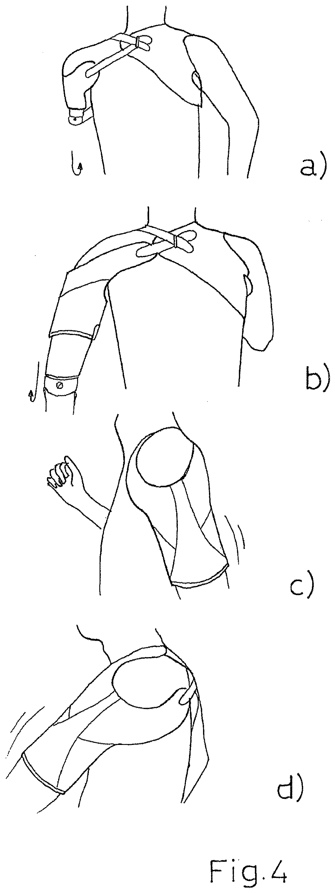

In den Figuren 4a - 4d sind verschiedene Bewegungszustände oder Bewegungsabschnitte bei der Benutzung der Prothesenschafthaltevorrichtung gezeigt. In der Figur 4a ist die Armprothese nach vorne geschwungen, dies führt dazu, dass der vordere Fortsatz 7 in Richtung auf das Schlüsselbein bewegt wird. Korrespondierend wird der dorsale Fortsatz 6 von der Führung 10 weg bewegt. Diese Bewegung führt dazu, dass das Kopplungselement 9 innerhalb der Führung 10 entlanggleitet und die freie Beweglichkeit der Prothese gewährleistet.

Figur 4b zeigt die Prothesenschafthaltevorrichtung in einem Zustand, bei der der Ellenbogen oder Arm nach hinten geschwungen ist, der dorsale Fortsatz 6 wird dadurch in Richtung auf die Führung 10 verlagert, das Kopplungselement 9 gleitet innerhalb der Führung 10 entlang, so dass der vordere Fortsatz 7 trotz der Verbindung mit dem dorsalen Fortsatz 6 sich von dem Schlüsselbein entfernen kann.

Die Position gemäß Figur 4b ist in der Seitenansicht in der Figur 4c gezeigt, die Position gemäß Figur 4a ist in der Seitenansicht in der Figur 4d dargestellt. Es ist deutlich zu erkennen, dass der dorsale Fortsatz 6 bei einer Bewegung in Schultergelenk nach vorne geführt wird, während der frontale Fortsatz 7 über das Schulterblatt in Richtung auf die Führung 10 bewegt wird. Die Bewegung der frontalen und dorsalen Fortsätze 6, 7 wird durch das Kopplungselement 9 und die nahezu feste Position der Führung 10 im Bereich der Wirbelsäule fixiert. Die Führung 10 ist an einer Stelle positioniert, bei der keine oder nur eine geringe Verlagerung der Führung 10 relativ zu dem Torso 1 zu erwarten ist, da sie ungefähr im Übergang von der Halswirbelsäule zur Brustwirbelsäule oder im oberen Drittel der Brustwirbelsäule positioniert ist.

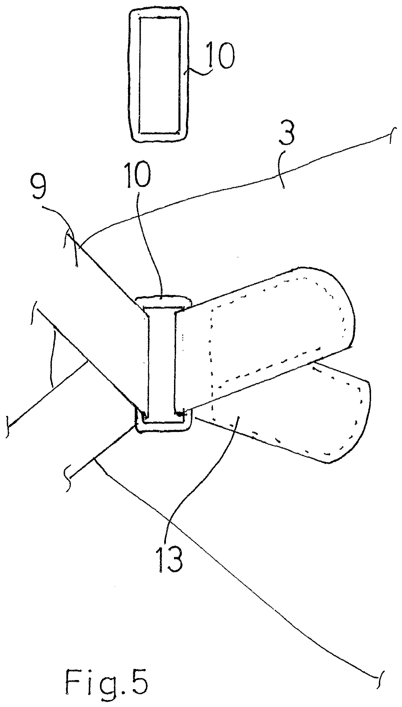

Figur 5 zeigt eine Detaildarstellung des Sicherungsteils 2 mit dem Rückenabschnitt 3 und der dort befestigten Führung 10. Im dargestellten Ausführungsbeispiel ist die Führung 10 als viereckiges Drahtelement mit einem im Wesentlichen geschlossenen Querschnitt ausgebildet. Über eine Gewebelasche 13 ist die Führung 10 klappbar aber im Wesentlichen ortsstabil an dem Sicherungsteil 2 fixiert. Das textile Element 13 kann angeklebt oder angenäht oder angeschweißt sein. Grundsätzlich ist es auch möglich, die Position der Führung 10 auf dem Sicherungsteil 2 variabel zu gestalten, um eine Anpassung an den Patienten zu ermöglichen.

Das Kopplungselement 9 ist um einen freien Schenkel der Führung 10 herum gelegt und kann in einer Gleitführung daran entlang bewegt werden, so dass ein Längenausgleich und eine freie Beweglichkeit des Kopplungselementes 9 gewährleistet ist. Die obere Darstellung zeigt die Form der Führung 10 in einer Einzeldarstellung.

Alternativ zu einem separaten Führungselement 10 aus einem Draht oder einem Kunststoff kann die Führung 10 auch innerhalb des Sicherungsteils 2 durch zwei Schlitze ausgebildet sein, durch die das Kopplungselement 9 hindurchgeführt wird. Die Ausgestaltung mit zwei Schlitzen hat den Vorteil, dass das Kopplungselement 9 überwiegend an der Oberseite und auf der Oberfläche des Sicherungsteils 2 entlanggeführt wird, um eine scheuernde Bewegung auf der Haut oder einem Gewebe weitestgehend zu vermeiden.

Die erfindungsgemäße Prothesenhaltevorrichtung legt über das Kopplungselement den Prothesenschaft 4 mechanisch über das Sicherungsteil 2 an dem Torso 1 des Prothesennutzers fest. Aufgrund der Tatsache, dass im frontalen Brustbereich nur relativ schmale Gurte oder gurtähnliche Abschnitte entlanggeführt sind und das Kopplungselement 9 auf der Rückseite in der Führung 10 umgelenkt wird, vorteilhafterweise im Bereich der oberen Brustwirbelsäule, wird das physiologische Gangbild des Anwenders nicht beeinträchtigt, da die Bewegung in den Schultergelenken nicht eingeschränkt wird. Zudem wir das Mitschwingen des Prothesenarmes ermöglicht und der kontralaterale, nicht versorgte Arm in der Beweglichkeit nicht eingeschränkt. Die gesamte Prothesenhaltevorrichtung ist von dem Prothesenschaft 4 abnehmbar, sie lässt sich somit waschen und leicht reinigen. Das Sicherungsteil 2 kann von dem übrigen, der Prothese zugeordneten Teil der Prothesenhaltevorrichtung getrennt werden, indem das Kopplungselement 9 von der Manschette oder der Hülse 5 mit seinen dorsalen oder frontalen Fortsätzen 6, 7 gelöst wird. Durch diesen modularen Aufbau ist es möglich, an unterschiedliche Prothesenschäfte 4 angepasste, vorgefertigte Hülsen 5 oder Manschetten zu fertigen und diese mit ebenfalls vorkonfektionierten Sicherungsteilen 2 frei zu kombinieren, je nachdem, welche Ausgestaltung dem jeweiligen Patienten am besten passt.

Grundsätzlich ist es auch möglich, dass das Kopplungselement 9 unmittelbar an dem Prothesenschaft 4 fixiert wird, also keine äußere Befestigungshülse 5 oder Manschette vorhanden ist, die auf der Außenseite den Prothesenschaft 4 vollständig umgibt. Vielmehr kann das Kopplungselement 9 über Formschlusselemente oder Klettverschlüsse direkt an dem Prothesenschaft 4 fixiert werden, gegebenenfalls in einer Schutzhülse 12 oder auf einer Schutzauflage, um ein unmittelbares Scheuern des beweglichen Kopplungselementes 9 auf der Haut zu vermeiden. Nach Möglichkeit ist das Sicherungsteil 2 so großflächig ausgebildet, dass keine oder nahezu keine direkte Berührung des Kopplungselementes 9 auf der Haut, insbesondere im Bereich des Schlüsselbeines auf der versorgten Seite erfolgt.

Durch die Gurtführung, also die Gleitführung des Kopplungselementes 9, im Rückenbereich wird das Mitschwingen des Prothesenarmes beim Gehen begünstigt. Die Prothesenschafthaltevorrichtung ist für den Patienten eigenständig anziehbar und ablegbar und kann durch einfache Maßnahmen an unterschiedliche Prothesenschäfte 4 angepasst werden. Das Material sowohl des Sicherungsteils 2 als auch der Manschette 5 mit den frontalen und dorsalen Fortsätzen 6, 7 kann ein textiles Material, beispielsweise unter Verwendung eines 3D-Abstandsgewirkes sein, ebenso sind Schaummaterialien, beispielsweise offenporige Schäume einsetztbar, um einen hohen Tragekomfort bei einer Minimierung der Feuchtigkeitsstauung zu gewährleisten.

## Patentansprüche

1. Prothesenschafthaltevorrichtung zur Sicherung eines Prothesenschaftes (4) an einer oberen Extremität, mit einem Sicherungsteil (2), das an dem Torso (1) unterhalb der Axilla der kontralateralen, unversorgten Seite eines Patienten entlangführbar ist, und mit einem Kopplungselement (9), das an dem Prothesenschaft (4) festlegbar und mit dem Sicherungsteil (2) verbindbar ist, **dadurch gekennzeichnet, dass** das Kopplungselement (9) verschieblich an dem Sicherungsteil (2) gelagert ist.

2. Prothesenschafthaltevorrichtung nach Anspruch 1, **dadurch gekennzeichnet, dass** das Kopplungselement (9) lösbar an dem Prothesenschaft (4) befestigbar ist.

3. Prothesenschafthaltevorrichtung nach Anspruch 1 oder 2, **dadurch gekennzeichnet, dass** das Kopplungselement (9) formschlüssig an dem Prothesenschaft (4) befestigt oder an einer Manschette oder Hülse (5) angeordnet ist, die an dem Prothesenschaft (4) lösbar befestigbar ist.

4. Prothesenschafthaltevorrichtung nach einem der voranstehenden Ansprüche, **dadurch gekennzeichnet, dass** das Kopplungselement (9) mehrteilig ausgebildet ist.

5. Prothesenschafthaltevorrichtung nach einem der voranstehenden Ansprüche, **dadurch gekennzeichnet, dass** das Kopplungselement (9) ein flexibles Zugelement aufweist, das beweglich in einer an dem Sicherungsteil (2) festgelegten oder ausgebildeten Führung (10) geführt ist.

6. Prothesenschafthaltevorrichtung nach einem der voranstehenden Ansprüche, **dadurch gekennzeichnet, dass** das Kopplungselement (9) in einer Schutzhülse (12) oder auf einer Schutzauflage verschieblich geführt ist, wobei die Schutzhülse (12) oder Schutzauflage im angelegten Zustand in dem Torso (1) anliegt.

7. Prothesenschafthaltevorrichtung nach einem der voranstehenden Ansprüche, **dadurch gekennzeichnet, dass** Kopplungselement (9) im angelegten Zustand an der Vorderseite und der Rückseite der versorgten Schulter entlanggeführt ist.

8. Prothesenschafthaltevorrichtung nach einem der voranstehenden Ansprüche, **dadurch gekennzeichnet, dass** das Sicherungsteil (2) als flächiger Zuschnitt ausgebildet ist und eine Öffnung (14) als Armdurchführung aufweist oder als Schlaufe mit endseitigen Verbindungselementen ausgebildet ist.

9. Prothesenschafthaltevorrichtung nach einem der voranstehenden Ansprüche, **dadurch gekennzeichnet, dass** das Sicherungsteil (2) eine Polsterung (15) im Axillabereich aufweist.

10. Prothesenschafthaltevorrichtung nach einem der voranstehenden Ansprüche, **dadurch gekennzeichnet, dass** das Sicherungsteil (2) im angelegten Zustand dorsal über die Medianebene in Richtung auf den Prothesenschaft (4) hinausragt.

11. Prothesenschafthaltevorrichtung nach einem der voranstehenden Ansprüche, **dadurch gekennzeichnet, dass** das Sicherungsteil (2) und/oder das Kopplungselement (9) aus einem textilen Werkstoff, Abstandsgewirk und/oder Schaummaterial gefertigt ist.

12. Prothesenschafthaltevorrichtung nach einem der voranstehenden Ansprüche, **dadurch gekennzeichnet, dass** das Kopplungselement (9) als Schlaufe ausgebildet ist.

13. System aus einer Prothesenschafthaltevorrichtung nach einem der voranstehenden Ansprüche und einem Prothesenschaft.

## Claims

1. Prosthesis socket retainer for securing a prosthesis socket (4) to an upper extremity, with a securing part (2) which can be guided along the torso (1) under the axilla of the contralateral, intact side of a patient, and with a coupling element (9) which can be fastened to the prosthesis socket (4) and can be connected to the securing part (2), **characterized in that** the coupling element (9) is mounted displaceably on the securing part (2).

2. Prosthesis socket retainer as claimed in claim 1, **characterized in that** the coupling element (9) can be secured releasably to the prosthesis socket (4).

3. Prosthesis socket retainer as claimed in claim 1 or 2, **characterized in that** the coupling element (9) is secured with form-fit engagement to the prosthesis socket (4) or is arranged on a cuff or sleeve (5) which can be secured releasably to the prosthesis socket (4).

4. Prosthesis socket retainer as claimed in one of the preceding claims, **characterized in that** the coupling element (9) has a multi-part design.

5. Prosthesis socket retainer as claimed in one of the preceding claims, **characterized in that** the coupling element (9) has a flexible tensioning element which is guided movably in a guide (10) that is fastened or formed on the securing part (2).

6. Prosthesis socket retainer as claimed in one of the preceding claims, **characterized in that** the coupling element (9) is guided displaceably in a protective sleeve (12) or on a protective support, wherein the protective sleeve (12) or protective support bears on the torso (1) in the fitted state.

7. Prosthesis socket retainer as claimed in one of the preceding claims, **characterized in that**, in the fitted state, the coupling element (9) is guided along the front aspect and the rear aspect of the treated shoulder.

8. Prosthesis socket retainer as claimed in one of the preceding claims, **characterized in that** the securing part (2) is designed as a planar blank and has an opening (14) as a passage for an arm or is configured as a loop with connecting elements at the ends.

9. Prosthesis socket retainer as claimed in one of the preceding claims, **characterized in that** the securing part (2) has a padding (15) in the axilla region.

10. Prosthesis socket retainer as claimed in one of the preceding claims, **characterized in that** the securing part (2), in the fitted state, extends dorsally beyond the median plane in the direction of the prosthesis socket (4).

11. Prosthesis socket retainer as claimed in one of the preceding claims, **characterized in that** the securing part (2) and/or the coupling element (9) is produced from a textile material, a spacer knit and/or foam material.

12. Prosthesis socket retainer as claimed in one of the preceding claims, **characterized in that** the coupling element (9) is configured as a loop.

13. System composed of a prosthesis socket retainer as claimed in one of the preceding claims and of a prosthesis socket.

## Revendications

1. Dispositif de retenue d'emboîture de prothèse pour bloquer une emboîture de prothèse (4) sur une extrémité supérieure, comportant une partie de blocage (2) susceptible d'être menée le long du torse (1) au-dessous de l'aisselle du côté controlatéral sain d'un patient, et comportant un élément de couplage (9) susceptible d'être immobilisé sur l'emboîture de prothèse (4) et d'être relié à la partie de blocage (2),
**caractérisé en ce que**
l'élément de couplage (9) est monté mobile en translation sur la partie de blocage (2).

2. Dispositif de retenue d'emboîture de prothèse selon la revendication 1,
**caractérisé en ce que**
l'élément de couplage (9) est susceptible d'être fixé de façon amovible à l'emboîture de prothèse (4).

3. Dispositif de retenue d'emboîture de prothèse selon la revendication 1 ou 2,
**caractérisé en ce que**
l'élément de couplage (9) est fixé en coopération de forme à l'emboîture de prothèse (4) ou à une manchette ou douille (5) qui est susceptible d'être fixée de façon amovible à l'emboîture de prothèse (4).

4. Dispositif de retenue d'emboîture de prothèse selon l'une des revendications précédentes,
**caractérisé en ce que**
l'élément de couplage (9) est réalisé en plusieurs parties.

5. Dispositif de retenue d'emboîture de prothèse selon l'une des revendications précédentes,
**caractérisé en ce que**
l'élément de couplage (9) présente un élément de traction flexible qui est guidé de façon mobile dans un guidage (10) immobilisé ou réalisé sur la partie de blocage (2).

6. Dispositif de retenue d'emboîture de prothèse selon l'une des revendications précédentes,
**caractérisé en ce que**
l'élément de couplage (9) est guidé de façon mobile en translation dans une douille de protection (12) ou sur un support de protection, la douille de protection (12) ou le support de protection prenant appui contre le torse (1), dans l'état appliqué.

7. Dispositif de retenue d'emboîture de prothèse selon l'une des revendications précédentes,
**caractérisé en ce que**
dans l'état appliqué, l'élément de couplage (9) est mené le long du côté avant et du côté arrière de l'épaule saine.

8. Dispositif de retenue d'emboîture de prothèse selon l'une des revendications précédentes,
**caractérisé en ce que**
la partie de blocage (2) est réalisé sous forme de flan surfacique et présente une ouverture (14) à titre de passage de bras ou à titre de boucle ayant des éléments de liaison du côté terminal.

9. Dispositif de retenue d'emboîture de prothèse selon l'une des revendications précédentes,
**caractérisé en ce que**
la partie de blocage (2) présente un rembourrage (15) dans la zone de l'aisselle.

10. Dispositif de retenue d'emboîture de prothèse selon l'une des revendications précédentes,
**caractérisé en ce que**
dans l'état appliqué, la partie de blocage (2) dépasse du côté dorsal au-delà du plan médian en direction de l'emboîture de prothèse (4).

11. Dispositif de retenue d'emboîture de prothèse selon l'une des revendications précédentes,
**caractérisé en ce que**
la partie de blocage (2) et/ou l'élément de couplage (9) est fabriqué(e) en un matériau textile, en un maillage d'écartement et/ou en un matériau de mousse.

12. Dispositif de retenue d'emboîture de prothèse selon l'une des revendications précédentes,
**caractérisé en ce que**
l'élément de couplage (9) est réalisé sous forme de boucle.

13. Système constitué d'un dispositif de retenue d'emboîture de prothèse selon l'une des revendications précédentes et d'une emboîture de prothèse.
